# EUROPEAN PATENT APPLICATION

(11) **EP 3 050 618 A1**
(43) Date of publication of application: **03.08.2016**
(21) Application number: 14847711.0
(22) Date of filing: 26.09.2014
(51) Int. Cl.: B01J 20/22, A61L 9/01, C07C 229/62, C07F 5/06, C07F 7/00, C07F 7/28

(54) **ALDEHYDE REMOVAL MATERIAL**

(30) Priority: 27.09.2013 JP 2013201258; 17.01.2014 JP 2014006638; 31.01.2014 JP 2014017182
(71) Applicant: Toyobo Co., Ltd., Osaka-shi Osaka 530-8230 (JP)
(72) Inventor: NISHIGUCHI, Yasuko, Otsu-shi Shiga 520-0292 (JP); MASUMORI, Tadao, Otsu-shi Shiga 520-0292 (JP); YOKOYAMA, Masayuki, Otsu-shi Shiga 520-0292 (JP); OKADA, Yuki, Otsu-shi Shiga 520-0292 (JP)
(74) Representative: Vossius & Partner Patentanwälte Rechtsanwälte mbB
(86) International application number: PCT/JP2014/075606
(87) International publication number: WO 2015/046417

(57) **Abstract**

To provide an aldehyde removing agent that can maintain high gas removal performance over a long period of time in the temperature and humidity range for normal life activities and can also have a reduced environmental impact. The problem is solved by using an NH group-containing compound-carrying porous metal complex or a porous metal complex including an NH group-containing organic ligand. For example, the porous metal complex includes at least one metal selected from elements belonging to Groups 2, 4, and 7 to 14 of the periodic table.

## Description

### TECHNICAL FIELD

The invention relates to an aldehyde removing agent.

### BACKGROUND ART

Air cleaners and deodorizers have been widely used mainly to remove cigarette smell from building rooms, car interiors, and other spaces. They are aimed at adsorbing and removing acetaldehyde, a main component of cigarette smoke, or causal substances of sick house, such as formaldehyde. Many removing agents have been examined for such purposes.

In general, porous materials are used as gas removing agents. Porous materials with narrow pore size distribution are used in order to improve selectivity, and porous materials with large specific surface area are used in order to provide high adsorption capacity. Examples of porous materials include activated carbon, zeolite, activated carbon fibers, and organic metal complexes or porous metal complexes (also called porous coordination polymers or metal organic frameworks), which are composed of metal ions and organic ligands and have received attention in recent years.

In particular, activated carbon has been known for a long time as an adsorbent for various organic substances. Unfortunately, activated carbon cannot sufficiently adsorb highly-polar, low-molecular-weight organic substances (such as acetaldehyde and formaldehyde). For the applications mentioned above, therefore, amine-carrying activated carbon with improved adsorption capacity is used.

Examples of disclosures on such amine-carrying materials include materials produced with aniline (PTD 1) and materials produced with ethanolamine (PTD 2).

Unfortunately, in the amine-carrying activated carbon, the pore surface of activated carbon is active and tends to deactivate or decompose the carried amine. In addition, amines with higher reactivity to adsorbed gas are more likely to be deactivated. Therefore, only amines with low reactivity can be used on activated carbon, which raises a problem in that it is difficult for amine-carrying activated carbon to provide high removal performance or maintain high removal performance over a long period of time.

As mentioned above, at present, there is no removing agent capable of maintaining aldehyde removal performance over a long period of time in the temperature and humidity range for normal life activities. As used herein, the term "the temperature and humidity range for normal life activities" refers to a temperature range of about -30 to about 50°C and a humidity range of about 20 to about 95%RH. As used herein, the term "aldehyde" is intended to include any of aliphatic, alicyclic, and aromatic aldehyde compounds. In particular, the removal of lower aliphatic aldehyde compounds such as formaldehyde and acetaldehyde often becomes an issue.

Activated carbon and inorganic oxides such as alumina, used as carriers as mentioned above, require a high-temperature manufacturing process at about 600 to 1,100°C, which consumes a huge amount of energy, results in only a weight yield of 50% or less depending on the manufacturing conditions, and emits a large amount of carbon dioxide. Therefore, such high environmental impact materials are required to be replaced with environmentally friendly materials.

Thus, there is disclosed a material for treating aldehyde compound-containing gas, which includes an organic metal complex having a one- or three-dimensional channel structure (PTD 3). The organic metal complex, which can be produced at about 200°C or lower, is a low environmental impact material. However, this treatment material has a problem in that it cannot have sufficient removal performance because it adsorbs an aldehyde compound through coordinate bonding between the metal ion and the carbonyl group of the aldehyde compound and thus preferentially adsorbs water molecules at the metal ion site in the temperature and humidity range for normal life activities.

### CITATION LIST

### PATENT DOCUMENT

PTD 1: Japanese Patent Laying-Open No. 56-53744
PTD 2: Japanese Patent Laying-Open No. 60-202735
PTD 3: Japanese Patent Laying-Open No. 09-323015

### SUMMARY OF INVENTION

### TECHNICAL PROBLEMS

It is an object of the invention to provide an aldehyde removing agent that can maintain high gas removal performance over a long period of time in the temperature and humidity range for normal life activities and can also have a reduced environmental impact.

### SOLUTIONS TO PROBLEMS

As a result of intensive studies to solve the problems, the inventors have accomplished the invention. Specifically, the invention is directed to the following.
1. An aldehyde removing agent including a porous metal complex including a metal and an organic ligand, wherein the organic ligand contains an NH group.
2. The aldehyde removing agent according to item 1, wherein the porous metal complex includes at least one metal selected from elements belonging to Groups 2, 4, and 7 to 14 of the periodic table.
3. The aldehyde removing agent according to item 1 or 2, wherein the porous metal complex includes at least one organic ligand containing a primary amino group, a secondary amino group, or an imino group.
4. The aldehyde removing agent according to any one of items 1 to 3, wherein the porous metal complex includes at least one metal selected from elements belonging to Groups 2, 4, and 7 to 14 of the periodic table and includes one organic ligand containing a primary amino group, a secondary amino group, or an imino group.
5. The aldehyde removing agent according to any one of items 1 to 4, wherein the metal is at least one of Al, Ti, and Zr, and the organic ligand is at least one of 2-aminoterephthalic acid and 2,5-diaminoterephthalic acid.
6. An aldehyde removing agent including: a porous metal complex including a metal and an organic ligand; and an NH group-containing compound carried in the porous metal complex.
7. The aldehyde removing agent according to item 6, wherein the NH group-containing compound carried in the porous metal complex contains a hydrazide group, a primary amino group, a secondary amino group, or an imino group.
8. The aldehyde removing agent according to item 6 or 7, wherein the organic ligand of the porous metal complex contains an NH group.
9. The aldehyde removing agent according to any one of items 6 to 8, wherein the porous metal complex includes at least one metal selected from elements belonging to Groups 2, 4, and 7 to 14 of the periodic table.
10. The aldehyde removing agent according to any one of items 6 to 9, wherein the porous metal complex includes at least one organic ligand containing a primary amino group, a secondary amino group, or an imino group.
11. The aldehyde removing agent according to any one of items 6 to 10,
   wherein the porous metal complex includes at least one metal selected from elements belonging to Groups 2, 4, and 7 to 14 of the periodic table and includes one organic ligand containing a primary amino group, a secondary amino group, or an imino group.
12. The aldehyde removing agent according to any one of items 6 to 11,
   wherein the metal is at least one of Ti, Zr, Mn, Fe, Co, Ni, Cu, Zn, Al, and Si, and the organic ligand is at least one of 2-aminoterephthalic acid and 2,5-diaminoterephthalic acid.

### ADVANTAGEOUS EFFECTS OF INVENTION

The aldehyde removing agent of the invention, which includes an NH group-containing compound-carrying porous metal complex or includes a porous metal complex having an NH group-containing organic ligand, can maintain high gas removal performance over a long period of time in the temperature and humidity range for normal life activities and can also have a reduced environmental impact. When the NH group-containing compound-carrying porous metal complex has an NH group-containing organic ligand in the porous metal complex, it has an increased amount of effective NH groups and thus has higher gas removal performance.

### BRIEF DESCRIPTION OF DRAWINGS

Fig. 1 is an X-ray diffraction pattern obtained by the power X-ray diffraction measurement of a metal complex obtained in Example 1.
Fig. 2 is an X-ray diffraction pattern obtained by the power X-ray diffraction measurement of a metal complex obtained in Example 2.
Fig. 3 is an X-ray diffraction pattern obtained by the power X-ray diffraction measurement of a metal complex obtained in Example 3.
Fig. 4 is an X-ray diffraction pattern obtained by the power X-ray diffraction measurement of a metal complex obtained in Example 4.
Fig. 5 is an X-ray diffraction pattern obtained by the power X-ray diffraction measurement of a metal complex obtained in Example 5.
Fig. 6 is an X-ray diffraction pattern obtained by the power X-ray diffraction measurement of a metal complex obtained in Example 6.

### DESCRIPTION OF EMBODIMENTS

Hereinafter, the invention will be described in detail.

The invention is directed to an agent for removing aldehydes by adsorption that occurs when the agent is brought into contact with aldehyde-containing gas being treated. The removing agent according to the invention includes a porous metal complex. The use of a porous metal complex is preferred because it can provide high adsorption capacity, prevent the deactivation of the carried amine, and reduce the environmental impact during the manufacture of the removing agent. The porous metal complex is preferably one of (1) a porous metal complex carrying an NH group-containing compound or (2) a porous metal complex including an NH group-containing organic ligand.

In the case (1), the porous metal complex can be free of such an active site as in activated carbon, so that the carried NH group-containing compound can be prevented from being deactivated. Therefore, even an NH group-containing compound highly reactive with aldehyde gas can be carried, which makes it possible to maintain high gas removal performance over a long period of time. The porous metal complex may include any organic ligand. Preferably, the porous metal complex includes at least one organic ligand having an NH group in order to remove aldehyde gas at higher efficiency.

In the case (2), the organic ligand as a component of the porous metal complex preferably includes at least one organic ligand having an NH group for high-efficiency removal of aldehyde gas. In the organic ligand, the NH group is fixed by covalent bonding, and therefore the NH group as a site reactive with aldehyde gas resists deactivation and decomposition, which makes it possible to maintain high gas removal performance over a long period of time.

In all the cases (1) and (2), therefore, an NH group is necessary for the adsorption of aldehyde gas through strong interaction with aldehyde gas or formation of an imine bond. A method of using an NH group-containing organic ligand to synthesize the porous metal complex is a good simple method for introducing the NH group into the organic ligand. Alternatively, after the synthesis of a porous metal complex, the organic ligand may be modified with an NH group-containing functional group.

The metal used to form the porous metal complex is preferably a metal belonging to Groups 2, 4, and 7 to 14 of the periodic table. Preferred examples include Mg, Ca, Sr, and Ba as Group 2 elements, Ti and Zr as Group 4 elements, Mn and Re as Group 7 elements, Fe, Ru, and Os as Group 8 elements, Co, Rh, and Ir as Group 9 elements, Ni, Pd, and Pt as Group 10 elements, Cu, Ag, and Au as Group 11 elements, Zn, Cd, and Hg as Group 12 elements, Al, Ga, In, and Tl as Group 13 elements, and B, Si, Ge, Sn, and Pb as Group 14 elements. Elements belonging to Groups 4 and 7 to 14 are more preferred. In particular, Ti, Zr, Mn, Fe, Co, Ni, Cu, Zn, Al, and Si are more preferably used in the invention. The use of Al, Ti, Zr, and Zn is most suitable because these metals can form chemically and hygrothermally stable compounds during the formation of the porous metal complex and has low influence on the environment.

The organic ligand of the porous metal complex is preferably bidentate or polydentate for skeleton construction. The organic ligand is more preferably an NH group-containing organic ligand for the purpose of high-efficiency removal of aldehyde gas. It is preferable to use at least one organic ligand containing a primary or secondary amino group or an imino group as an NH group. In addition, a mixture of an NH group-free organic ligand and an NH group-containing organic ligand may also be used. Examples of an organic ligand containing a primary amino group as an NH group and capable of bidentate or polydentate coordination include dicarboxylic acids and derivatives thereof, such as 2-aminoterephthalic acid, 2,5-diaminoterephthalic acid, 5-aminoisophthalic acid hydrate, 2-amino(1,1'-biphenyl)-4,4'-dicarboxylic acid, 3-amino(1,1'-biphenyl)-4,4'-dicarboxylic acid, 4-amino(1,1'-biphenyl)-3,4'-dicarboxylic acid, and 3',4-diamino(1, l'-biphenyl)-3,4'-dicarboxylic acid; tricarboxylic acids and derivatives thereof, such as 2-amino-1,3,5-benzenetricarboxylic acid and 2,4-diamino-1,3,5-benzenetricarboxylic acid; and heterocyclic compounds and derivatives thereof, such as 2-aminoimidazole sulfate, 2-aminomethylimidazole dihydrochloride, 2-aminobenzimidazole, adenine, 2-aminopurine, 3-amino-5-methyl-4H-1,2,4-triazole, 2-aminopyrazine, 2-aminopyrimidine, 4-aminopyrimidine, 5-aminopyrimidine, 2,4-diaminopyrimidine, 2,4,6-triaminopyrimidine, melamine, 2,4-diamino-6-methyltriazine, 3-aminopyrazine-2-carboxylic acid, and 2-aminoisonicotinic acid. Examples of an organic ligand containing a secondary amino group as an NH group and capable of bidentate or polydentate coordination include 2-methylaminoterephthalic acid, 2-ethylaminoterephthalic acid, 2-propylaminoterephthalic acid, 2,5-bis(methylamino)-terephthalic acid, 5-methylaminoisophthalic acid, 5-ethylaminoisophthalic acid, 5-propylaminoisophthalic acid, 4-methylaminoisophthalic acid, 4-ethylaminoisophthalic acid,
4-propylaminoisophthalic acid, 2-phenylaminotrimesic acid, 2-methylaminoimidazole, 2-ethylaminoimidazole, 5-methylaminoimidazole, 5-ethylaminoimidazole, 2-methylaminobenzimidazole, 3-methylamino-4H-1,2,4-triazole, 3-ethylamino-4H-1,2,4-triazole, 2-methylaminopyrazine,
2,3-bis(methylamino)-pyrazine, 2,6-bis(methylamino)-pyrazine, 2-methylaminopyrimidine, 4-methylaminopyrimidine, 5-methylaminopyrimidine, 2,4-bis(methylamino)-pyrimidine, 2,4,6-tris(methylamino)-pyrimidine, 2,4,6-tris(methylamino)-triazine, and 2-methylaminoisonicotinic acid. Examples of an organic ligand containing an imino group as an NH group and capable of bidentate or polydentate coordination include 2-ethylidene-amino-terephthalic acid, 5-(aminoiminomethyl)aminoisophthalic acid,
N,N-dimethyl-N'-(2-methyl-1H-imidazol-5-yl)-methaneimidamide, N,N-dimethyl-N'-1H-1,2,4-triazol-5-yl-methaneimidamide, and 2-methyleneamino-4,6-diaminotriazine. Examples of an NH group-free organic ligand capable of bidentate or polydentate coordination include dicarboxylic acids and derivatives thereof, such as terephthalic acid, isophthalic acid,
2,5-dihydroxyterephthalic acid, 1,4-naphthalenedicarboxylic acid, 2,6-naphthalenedicarboxylic acid, 4,4'-biphenyldicarboxylic acid, 3,3'-biphenyldicarboxylic acid, fumaric acid, malonic acid, and adipic acid; tricarboxylic acids and derivatives thereof, such as biphenyl-3,4'-5-tricarboxylic acid, 1,3,5-tris(4'-carboxy[1,1'-biphenyl]-4-yl)benzene, 1,3,5-tris(4-carboxyphenyl)benzene, and 1,3,5-benzenetricarboxylic acid; tetracarboxylic acids and derivatives thereof, such as p-terphenyl-3,3',5,5'-tetracarboxylic acid (also called
5,5'-(1,4-phenylene)bisisophthalic acid) and 1,2,4,5-tetrakis(4-carboxyphenyl)benzene; imidazoles and derivatives thereof, such as imidazole, 2-methylimidazole, and 2-phenylimidazole; pyrazoles and derivatives thereof, such as 4,4'-bipyrazolate, 3,3',5,5'-tetramethyl-4,4'-bipyrazolate, and 1,3,5-tris(1H-1,2-pyrazol-4-yl)benzene; triazoles and derivatives thereof, such as 1,3,5-tris(1H-1,2,3-triazol-5-yl)benzene; tetrazoles and derivatives thereof, such as 5,5'-bistetrazole, 5,5'-azobis-1H-tetrazole, and 1,3,5-tris(2H-tetrazol-5-yl)benzene; pyridines and derivatives thereof, such as 1,2-bis(4-pyridyl)ethane and 4,4'-bipyridine; and triethylenediamine, pyrazine, and piperazine. In particular, the invention preferably uses 2-aminoterephthalic acid or 2,5-diaminoterephthalic acid so that the number of NH groups per unit weight of the porous metal complex can be high during the formation of the porous metal complex.

The metal complex can be produced by allowing a metal and an organic ligand to react at about -10°C to about 200°C for 120 hours or less. The metal complex is preferably produced by reaction at 0°C to about 170°C for 48 hours or less, more preferably by reaction at 25°C to 150°C for 24 hours. The reaction is preferably performed at a lower temperature for a shorter time, so that thermal energy can be saved.

A solution used to produce the metal complex preferably has a metal salt molar concentration of 0.005 to 5.0 mol/L, more preferably 0.01 to 2.0 mol/L. The desired metal complex can be obtained by reaction at a concentration lower than the range, but such a lower concentration can lead to a lower yield and thus is not preferred. If the concentration is higher than the range, part of the metal salt may remain unreacted, which can make it difficult to purify the resulting metal complex.

A solution used to produce the metal complex preferably has an organic ligand molar concentration of 0.001 to 5.0 mol/L, more preferably 0.005 to 2.0 mol/L. The desired metal complex can be obtained by reaction at a concentration lower than the range, but such a lower concentration can lead to a lower yield and thus is not preferred. If the concentration is higher than the range, the solubility may decrease, and the reaction may fail to proceed smoothly.

The solvent for use in the production of the metal complex may be water, methanol, ethanol, dimethylformamide (DMF), diethylformamide (DEF), dimethylacetamide (DMAc), tetrahydrofuran (THF), or a mixed solvent of any combination thereof. The use of water is preferred in view of environmental impact reduction.

In the invention, the NH group-containing compound to be carried preferably has a hydrazide group, a primary or secondary amino group, or an imino group, and more preferably has a hydrazide group in order to have higher aldehyde-removal performance.

The compound having a hydrazide group as an NH group may be a monohydrazide compound having one hydrazide group per molecule, a dihydrazide compound having two hydrazide groups per molecule, or a polyhydrazide compound having three or more hydrazide groups per molecule.

A specific example of the monohydrazide compound is represented by, for example, formula (1): R-CO-NHNH2, wherein R represents a hydrogen atom, an alkyl group, or an aryl group optionally having a substituent.

In formula (1), the alkyl group represented by R may be linear alkyl of 1 to 12 carbon atoms, such as methyl, ethyl, n-propyl, n-butyl, n-pentyl, n-hexyl, n-heptyl, n-octyl, n-nonyl, n-decyl, or n-undecyl. The aryl group may be, for example, phenyl, biphenyl, or naphthyl, among which phenyl is preferred. The aryl group may have a substituent, which may be, for example, hydroxyl, a halogen atom such as fluorine, chlorine, or bromine, or linear or branched alkyl of 1 to 4 carbon atoms, such as methyl, ethyl, n-propyl, isopropyl, n-butyl, tert-butyl, or isobutyl.

More specifically, examples of the hydrazide compound of formula (1) include lauric acid hydrazide, salicylic acid hydrazide, formhydrazide, acetohydrazide, propionic acid hydrazide, p-hydroxybenzoic acid hydrazide, naphthoic acid hydrazide, and 3-hydroxy-2-naphthoic acid hydrazide.

An example of the dihydrazide compound is represented by, for example, formula (2): H2NHN-X-NHNH2, wherein X represents a -CO- or -CO-A-CO- group, wherein A represents an alkylene or arylene group.

The alkylene group represented by A in formula (2) may be, for example, linear alkylene of 1 to 12 carbon atoms, such as methylene, ethylene, trimethylene, tetramethylene, pentamethylene, hexamethylene, heptamethylene, octamethylene, nonamethylene, decamethylene, or undecamethylene. The alkylene group may have a substituent, which may be, for example, hydroxyl. The arylene group may be, for example, phenylene, biphenylene, naphthylene, anthrylene, or phenanthrylene, among which phenylene and naphthylene are preferred. The arylene group may also have a substituent, which may be the same as for the aryl group.

Specifically, examples of the dihydrazide compound of formula (2) include carbohydrazide, oxalic acid dihydrazide, malonic acid dihydrazide, succinic acid dihydrazide, adipic acid dihydrazide, azelaic acid dihydrazide, sebacic acid dihydrazide, dodecane diacid dihydrazide, maleic acid dihydrazide, fumaric acid dihydrazide, diglycolic acid dihydrazide, tartaric acid dihydrazide, malic acid dihydrazide, isophthalic acid dihydrazide, terephthalic acid dihydrazide, dimer acid dihydrazide, 2,6-naphthoic acid dihydrazide, and other dibasic acid dihydrazides. The dihydrazide for use in the invention may also be any of various dibasic acid dihydrazide compounds, 2,4-dihydrazino-6-methylamino-sym-triazine, and other compounds shown in Japanese Patent Publication No. 02-4607.

Specifically, the polyhydrazide compound may be, for example, polyacrylic acid hydrazide. Among these compounds, dihydrazide compounds are preferred, and dibasic acid dihydrazides are particularly preferred. Adipic acid dihydrazide is more preferred.

Examples of a compound having a primary amino group as an NH group include methylamine, ethylamine, propanamine, ethanolamine, aniline, sulfanilic acid, and ethyl carbamate, and polybasic amines such as ethylenediamine, 1,3-propanediamine, 1,8-octanediamine, triethyltetramine, and polyethylene imine. The compound having a secondary amino group as an NH group may be, for example, dimethylamine, diethylamine, diethanolamine, pyrrolidine, pyrrole, or triazole. The compound having an imino group as an NH group may be, for example, benzophenone imine or guanidine.

Any method capable of imparting the desired properties may be used to allow the porous metal complex to carry the NH group-containing compound. For example, the porous metal complex is allowed to carry the NH group-containing compound through absorption by a method of spraying or applying a solvent solution of the NH group-containing compound onto the porous metal complex, which is followed by the step of removing the solvent by drying. It is conceivable that the NH group-containing compound is carried by coordination to the metal as a component of the porous metal complex without forming any covalent bond with the organic ligand. An appropriate solvent may be selected taking into account the properties of the NH group-containing compound and workability. In particular, an aqueous solvent is preferably used in view of safety and workability.

A more preferred method includes allowing a reagent solution to be absorbed and carried with stirring and mixing in a vessel before water accumulates. The use of this method makes it possible to reduce aggregation and adhesion in a solution where particles of the porous metal complex are crosslinked.

The amount of the carried NH group-containing compound is preferably from 1 to 100 parts by weight, more preferably from 1 to 50 parts by weight, even more preferably from 5 to 10 parts by weight, based on 100 parts by weight of the porous metal complex. If it is less than 1 part by weight, the capacity and the rate may be insufficient. If it is more than 100 parts by weight, pore clogging can significantly occur, which can make it impossible to obtain a certain adsorption capacity.

A gas adsorbent including the porous metal complex according to the invention, which can be widely used in standard homes and industrial fields, will be exposed to various service conditions and environments. Therefore, the gas adsorbent preferably has an aldehyde removal performance retention rate of 80% or more, more preferably 90% or more, after it is allowed to stand for 24 hours in an atmosphere at a temperature of 80°C and a relative humidity of 95%, which are conditions given taking into account long-term use and storage stability in the temperature and humidity range for normal life activities.

The gas removing agent obtained according to the invention has high aldehyde removal performance. Therefore, when used as a deodorizer, the gas removing agent can reduce the amount of aldehydes generated in building rooms and car interiors. These applications are non-limiting examples, and the gas removing agent can be widely used to reduce aldehydes in exhaust gases from cars and factories manufacturing chemicals and electric machinery and appliances. When carried on a filter or any other member, the gas removing agent of the invention can also be used as an aldehyde removing filter. When installed in an air cleaner, the aldehyde removing filter can easily reduce the amount of aldehydes in building rooms and car interiors. The aldehyde removing filter may also be placed in plants for chemical industries or plants for manufacturing electric machinery and appliances to control the emission of aldehydes into the atmosphere. The aldehyde removing filter may be in any shape. For example, a method of producing the aldehyde removing filter in a planer, pleated, or honeycomb shape is preferred. A pleated filter for use as a direct flow filter or a honeycomb-shaped filter for use as a parallel flow filter can have an increased area for contact with the gas to be treated, which can simultaneously improve the removal efficiency and reduce the pressure loss of the deodorizing filter.

### EXAMPLES

Hereinafter, the invention will be more specifically described with reference to examples. It will be understood that the examples below are not intended to limit the invention and modifications or alterations thereof may be made within a range meeting the gist described above and below, all of which are included in the technical scope of the invention. The analysis or evaluation in the examples and the comparative examples was performed as described below.

### <Powder X-ray diffraction measurement>

Whether a metal complex has a crystal structure in which an organic ligand coordinates to metal ions can be determined by, for example, powder X-ray diffraction measurement. The synthesized porous metal complexes were measured by symmetrical reflection method using a powder X-ray diffractometer (NEW D8 ADVANCE manufactured by Bruker AXS). The measurement conditions were as follows.
1) X-ray source: CuKα (λ = 1.5418 Å) 40 kV, 200 mA
2) Goniometer: Vertical goniometer
3) Detector: Scintillation counter
4) Diffraction angle (2θ) range: 3 to 90°
5) Scanning step: 0.05°
6) Integral time: 0.5 seconds/step
7) Slit: Divergence slit 0.5°, receiving slit 0.15 mm, scattering slit 0.5°

### <Nitrogen adsorption measurement>

### <Measurement of specific surface area and pore volume>

About 100 mg of a porous metal complex was sampled. After the sample was dried under vacuum at 120°C for 12 hours, the weight of the sample was measured. Using a high-performance, specific surface area-pore distribution analyzer (ASAP 2020 manufactured by Micromeritics Instrument Corporation), the adsorption isotherm curve of the sample was prepared by measuring the amount of adsorption of nitrogen gas at the boiling point (-195.8°C) of liquid nitrogen under 40 relative pressures, which were gradually increased in the range of 0.02 to 0.95. Using analysis software (ASAP 2020 V3.04 manufactured by Micromeritics Instrument Corporation), a BET plot was obtained from the results under relative pressures of 0.02 to 0.15, and the BET specific surface area (m²/g) per unit weight was determined from the plot. The total pore volume (cc/g) was also determined from the data under a relative pressure of 0.95.

### <Adsorption test using aldehyde gas flow system>

After a column was charged with 0.088 cc of a porous metal complex, a test gas was allowed to flow at 0.2 L/minute through the column. The gas concentration was measured at the inlet and outlet of the sample line at regular time intervals using a formaldehyde meter htV (manufactured by JMS Co., Ltd.). The removal rate was calculated from the gas concentration ratio. The test was continued until the removal rate reached 50% or less. The removal rate curve was integrated with respect to the amount of formaldehyde supply (calculated from the concentration, flow rate, and temperature) to give the amount (mg) of adsorption of formaldehyde, which was divided by the weight of the sample to calculate the adsorption capacity (mg/g). Before use, the sample was dried under vacuum at 120°C for 12 hours so that the adsorbed substances were removed. The evaluation conditions are shown in detail below.
1) Measurement atmosphere: Air at 25°C and 50%RH
2) Pressure: Ambient pressure
3) Test gas composition: Formaldehyde at a concentration of 3 ppm (dilution with the air at 25°C and 50%RH)
4) Space velocity: 1,350,000 hr⁻¹
5) Average particle size: 1 to 5 µm

### <Measurement of aldehyde removal performance retention>

A porous metal complex was dried under vacuum at 120°C for 12 hours so that the adsorbed substances such as water were removed. The dried complex was placed in a sample tube and then allowed to stand for 24 hours in a thermo-hygrostat (LH33-12P manufactured by Nagano Science Co., Ltd.) at a temperature of 80°C and a relative humidity of 95%. Subsequently, the adsorption test using the aldehyde gas flow system was performed, in which the aldehyde removal performance retention was calculated from the following formula (i): aldehyde removal performance retention (%) = {(the adsorption capacity after the standing for 24 hours)/(the adsorption capacity before the standing for 24 hours)} x 100.

### <Example 1>

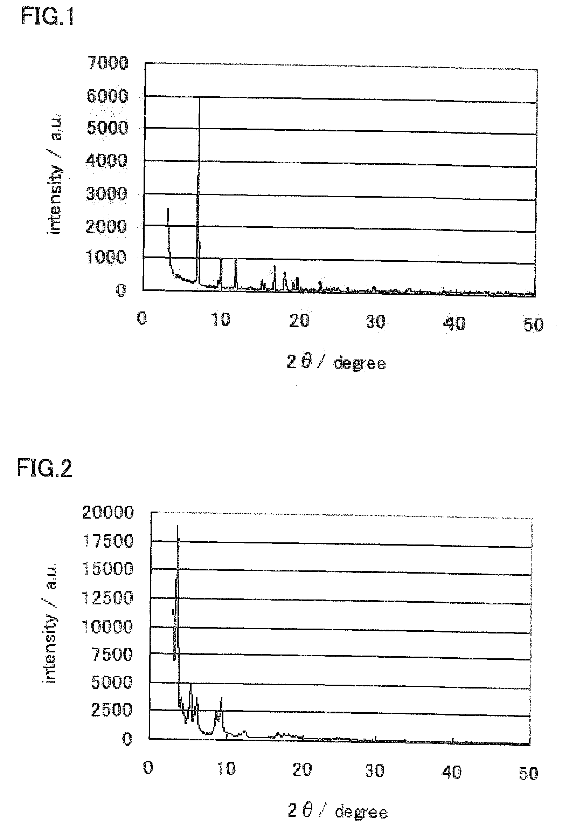

3.6 ml (12.3 mmol) of tetraisopropyl orthotitanate and 3.6 g (19.9 mmol) of 2-aminoterephthalic acid were dissolved in 48 ml of N,N-dimethylformamide and 12 ml of methanol and then subjected to synthesis at 150°C for 18 hours. The resulting porous metal complex was subjected to identification by powder X-ray diffraction measurement. As a result, the porous metal complex was found to be a crystalline material having an organic ligand in coordination with the metal ions. Fig. 1 shows the X-ray diffraction pattern obtained by the powder X-ray diffraction measurement. The physical properties of the product were evaluated by the nitrogen adsorption measurement. As a result, the product had a BET specific surface area of 1,426 m²/g and was found to have a porous structure. The product was then subjected to the adsorption test using the aldehyde gas flow system. Table 1 shows the results. After the product was allowed to stand in an atmosphere at a temperature of 80°C and a relative humidity of 95% for 24 hours, the product was also subjected to the adsorption test using the aldehyde gas flow system. The aldehyde removal performance retention was calculated to be 97%.

### <Example 2>

1.0 g (4.2 mmol) of aluminum chloride hexahydrate and 1.1 g (6.1 mmol) of 2-aminoterephthalic acid were dissolved in 60 ml of N,N-dimethylformamide and then subjected to synthesis at 130°C for 72 hours. The resulting porous metal complex was subjected to identification by powder X-ray diffraction measurement. As a result, the porous metal complex was found to be a crystalline material having an organic ligand in coordination with the metal ions. Fig. 2 shows the X-ray diffraction pattern obtained by the powder X-ray diffraction measurement. The physical properties of the product were evaluated by the nitrogen adsorption measurement. As a result, the product had a BET specific surface area of 784 m²/g and was found to have a porous structure. The product was then subjected to the adsorption test using the aldehyde gas flow system. Table 1 shows the results.

### <Example 3>

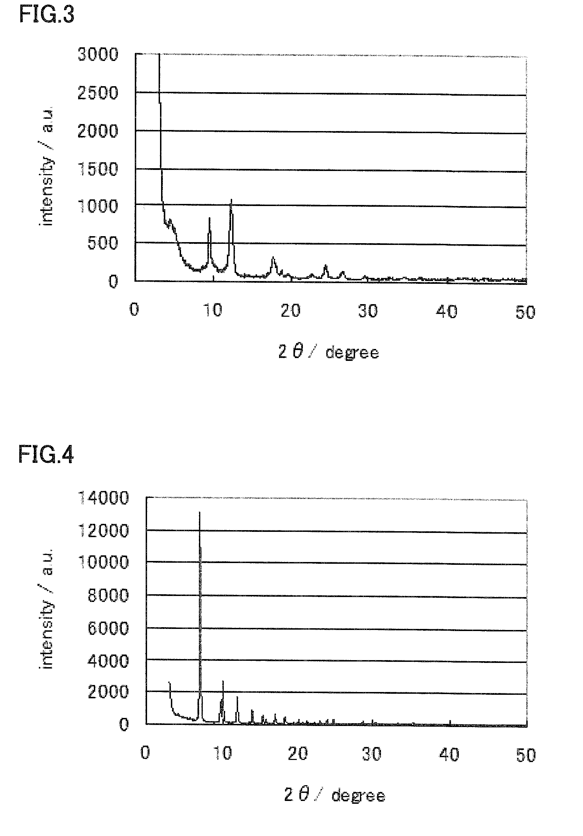

1.6 g (4.2 mmol) of aluminum nitrate nonahydrate and 1.1 g (6.1 mmol) of 2-aminoterephthalic acid were dissolved in 60 ml of N,N-dimethylformamide and then subjected to synthesis at 130°C for 72 hours. The resulting porous metal complex was subjected to identification by powder X-ray diffraction measurement. As a result, the porous metal complex was found to be a crystalline material having an organic ligand in coordination with the metal ions. Fig. 3 shows the X-ray diffraction pattern obtained by the powder X-ray diffraction measurement. The physical properties of the product were evaluated by the nitrogen adsorption measurement. As a result, the product had a BET specific surface area of 317 m²/g and was found to have a porous structure. The product was then subjected to the adsorption test using the aldehyde gas flow system. Table 1 shows the results.

### <Example 4>

1.2 g (4.9 mmol) of aluminum chloride hexahydrate and 0.3 g (1.7 mmol) of 2-aminoterephthalic acid were dissolved in 48 ml of methanol and then subjected to synthesis at 100°C for 48 hours. The resulting porous metal complex was subjected to identification by powder X-ray diffraction measurement. As a result, the porous metal complex was found to be a crystalline material having an organic ligand in coordination with the metal ions. Fig. 4 shows the X-ray diffraction pattern obtained by the powder X-ray diffraction measurement. The physical properties of the product were evaluated by the nitrogen adsorption measurement. As a result, the product had a BET specific surface area of 1,256 m²/g and was found to have a porous structure. The product was then subjected to the adsorption test using the aldehyde gas flow system. Table 1 shows the results.

### <Example 5>

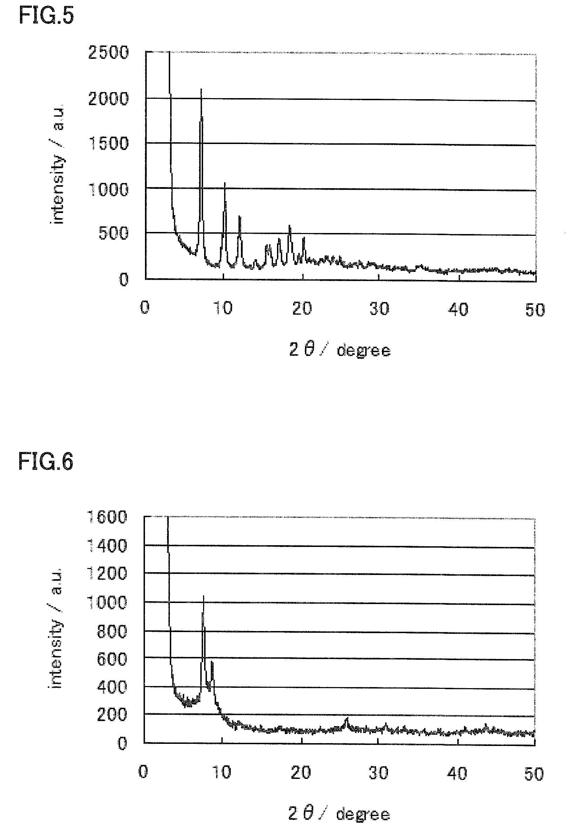

0.46 g (1.9 mmol) of aluminum chloride hexahydrate and 0.13 g (1.7 mmol) of 2,5-diaminoterephthalic acid were dissolved in 6 ml of methanol and then irradiated with microwaves at 145°C for 3 minutes. The resulting porous metal complex was subjected to identification by powder X-ray diffraction measurement. As a result, the porous metal complex was found to be a crystalline material having an organic ligand in coordination with the metal ions. Fig. 5 shows the X-ray diffraction pattern obtained by the powder X-ray diffraction measurement. The physical properties of the product were evaluated by the nitrogen adsorption measurement. As a result, the product had a BET specific surface area of 788 m²/g and was found to have a porous structure. The product was then subjected to the adsorption test using the aldehyde gas flow system. Table 1 shows the results.

### <Example 6>

1.3 g (4.9 mmol) of zirconium chloride and 0.9 g (5.0 mmol) of 2-aminoterephthalic acid were dissolved in 60 ml of N,N-dimethylformamide and then subjected to synthesis at 120°C for 24 hours. The resulting porous metal complex was subjected to identification by powder X-ray diffraction measurement. As a result, the porous metal complex was found to be a crystalline material having an organic ligand in coordination with the metal ions. Fig. 6 shows the X-ray diffraction pattern obtained by the powder X-ray diffraction measurement. The physical properties of the product were evaluated by the nitrogen adsorption measurement. As a result, the product had a BET specific surface area of 575 m²/g and was found to have a porous structure. The product was then subjected to the adsorption test using the aldehyde gas flow system. Table 1 shows the results.

### <Example 7>

3.6 ml (12.3 mmol) of tetraisopropyl orthotitanate and 3.6 g (19.9 mmol) of 2-aminoterephthalic acid were dissolved in 48 ml of N,N-dimethylformamide and 12 ml of methanol and then heated at 150°C for 18 hours to form a porous metal complex. The resulting porous metal complex was allowed to absorb an adipic acid dihydrazide aqueous solution and then dried under vacuum at 80°C. The adipic acid dihydrazide was carried in an amount of 1.0 part by weight by 100 parts by weight of the porous metal complex. After allowed to carry the adipic acid dihydrazide, the porous metal complex was subjected to physical properties evaluation by the nitrogen adsorption measurement. The product had a BET specific surface area of 1,401 m²/g. The product was then subjected to the adsorption test using the aldehyde gas flow system. Table 1 shows the results.

### <Example 8>

The same process was performed using the same porous metal complex as in Example 7, except that 100 parts by weight of the porous metal complex was allowed to carry 2.5 parts by weight of adipic acid dihydrazide. The product had a BET specific surface area of 1,371 m²/g. The product was then subjected to the adsorption test using the aldehyde gas flow system. Table 1 shows the results.

### <Example 9>

The same process was performed using the same porous metal complex as in Example 7, except that 100 parts by weight of the porous metal complex was allowed to carry 5.0 parts by weight of adipic acid dihydrazide. The product had a BET specific surface area of 1,326 m²/g. The product was then subjected to the adsorption test using the aldehyde gas flow system. Table 1 shows the results.

### <Example 10>

The same process was performed using the same porous metal complex as in Example 7, except that 100 parts by weight of the porous metal complex was allowed to carry 10 parts by weight of adipic acid dihydrazide. The product had a BET specific surface area of 1,066 m²/g. The product was then subjected to the adsorption test using the aldehyde gas flow system. Table 1 shows the results.

### <Example 11>

The same porous metal complex as used in Examples 7 to 10 was allowed to absorb an ethyl carbamate aqueous solution and then dried under vacuum at 80°C. The ethyl carbamate was carried in an amount of 5.0 parts by weight by 100 parts by weight of the porous metal complex. After allowed to carry the ethyl carbamate, the porous metal complex was subjected to physical properties evaluation by the nitrogen adsorption measurement. The product had a BET specific surface area of 1,176 m²/g. The product was then subjected to the adsorption test using the aldehyde gas flow system. Table 1 shows the results.

### <Example 12>

A porous metal complex (Basolite (registered trademark) Z1200 manufactured by BASF) produced with Zn and 2-methylimidazole was allowed to absorb an adipic acid dihydrazide aqueous solution and then dried under vacuum at 80°C. The adipic acid dihydrazide was carried in an amount of 10 parts by weight by 100 parts by weight of the porous metal complex. After allowed to carry the adipic acid dihydrazide, the porous metal complex was subjected to physical properties evaluation by the nitrogen adsorption measurement. The product had a BET specific surface area of 1,264 m²/g. The product was then subjected to the adsorption test using the aldehyde gas flow system. Table 1 shows the results.

### <Comparative Example 1>

A porous metal complex (Basolite (registered trademark) A100 manufactured by BASF) produced with Al and terephthalic acid was subjected to physical properties evaluation by the nitrogen adsorption measurement. The porous metal complex had a BET specific surface area of 575 m²/g and was confirmed to have a porous structure. The metal complex was then subjected to the adsorption test using the aldehyde gas flow system. Table 1 shows the results.

### <Comparative Example 2>

Coconut shell activated carbon (specific surface area: 1,450 m²/g, pore volume: 0.6 cc/g) was allowed to absorb a sulfanilic acid aqueous solution and then dried at 80°C. The sulfanilic acid was carried in an amount of 15 parts by weight by 100 parts by weight of the activated carbon. After allowed to carry the sulfanilic acid, the activated carbon was subjected to physical properties evaluation by the nitrogen adsorption measurement. The product had a BET specific surface area of 1,350 m²/g. The product was then subjected to the adsorption test using the aldehyde gas flow system. Table 1 shows the results. The coconut shell activated carbon was also allowed to carry adipic acid dihydrazide and other NH group-containing compounds, and then the resulting products were also subjected to the adsorption test using the aldehyde gas flow system. Among these products, the sulfanilic acid-carrying activated carbon showed the best performance.

**[Table 1]**

| | Metal | Organic ligand | Carried compound | Carried amount (wt%) | Adsorption capacity (mg/g) | Aldehyde removal performance retention(%) | BET specific surface area (m²/g) |
|---|---|---|---|---|---|---|---|
| Example 1 | Ti | 2-aminoterephthalic acid | - | - | 6.5 | 97 | 1426 |
| Example 2 | Al | 2-aminoterephthalic acid | - | - | 10.9 | 81 | 784 |
| Example 3 | Al | 2-aminoterephthalic acid | - | - | 3.0 | 41 | 317 |
| Example 4 | Al | 2-aminoterephthalic acid | - | - | 2.9 | 93 | 1256 |
| Example 5 | Al | 2,5-diaminoterephthalic acid | - | - | 12.2 | 99 | 788 |
| Example 6 | Zr | 2-aminoterephthalic acid | - | - | 12.5 | 47 | 575 |
| Example 7 | Ti | 2-aminoterephthalic acid | Adipic acid dihydrazide | 1 | 13 | - | 1401 |
| Example 8 | Ti | 2-aminoterephthalic acid | Adipic acid dihydrazide | 2.5 | 20.1 | - | 1371 |
| Example 9 | Ti | 2-aminoterephthalic acid | Adipic acid dihydrazide | 5 | 38 | 91 | 1326 |
| Example 10 | Ti | 2-aminoterephthalic acid | Adipic acid dihydrazide | 10 | 19.9 | - | 1066 |
| Example 11 | Ti | 2-aminoterephthalic acid | Ethyl carbamate | 5 | 9.4 | 80 | 1176 |
| Example 12 | Zn | 2-methylimidazole | Adipic acid dihydrazide | 10 | 15.7 | 62 | 1264 |
| Comparative Example 1 | Al | Terephthalic acid | - | - | 0.01 | 0 | 575 |
| Comparative Example 2 | Coconut shell activated carbon | | Sulfanilic acid | 15 | 1.8 | 22 | 1350 |

### INDUSTRIAL APPLICABILITY

The aldehyde removing agent of the invention can provide a more comfortable atmosphere by cleaning the air in rooms and cars. The aldehyde removing agent of the invention is expected to help reduce sick house syndrome and other disorders. In addition, the aldehyde removing agent of the invention can reduce the amounts of aldehydes released to the air and thus contribute to the reduction of environmental pollution. The porous metal complex, which can be used as an alternative to activated carbon, alumina, or other materials, is significantly effective in saving production energy and can significantly contribute to environmental impact reduction.

## Claims

1. An aldehyde removing agent comprising a porous metal complex comprising a metal and an organic ligand, wherein said organic ligand contains an NH group.

2. The aldehyde removing agent according to claim 1, wherein the porous metal complex comprises at least one metal selected from elements belonging to Groups 2, 4, and 7 to 14 of periodic table.

3. The aldehyde removing agent according to claim 1 or 2, wherein the porous metal complex comprises at least one organic ligand containing a primary amino group, a secondary amino group, or an imino group.

4. The aldehyde removing agent according to any one of claims 1 to 3,
wherein the porous metal complex comprises at least one metal selected from elements belonging to Groups 2, 4, and 7 to 14 of periodic table and comprises one organic ligand containing a primary amino group, a secondary amino group, or an imino group.

5. The aldehyde removing agent according to any one of claims 1 to 4,
wherein the metal is at least one of Al, Ti, and Zr, and said organic ligand is at least one of 2-aminoterephthalic acid and 2,5-diaminoterephthalic acid.

6. An aldehyde removing agent comprising:
a porous metal complex comprising a metal and an organic ligand; and
an NH group-containing compound carried in the porous metal complex.

7. The aldehyde removing agent according to claim 6, wherein the NH group-containing compound carried in the porous metal complex contains a hydrazide group, a primary amino group, a secondary amino group, or an imino group.

8. The aldehyde removing agent according to claim 6 or 7, wherein the organic ligand of the porous metal complex contains an NH group.

9. The aldehyde removing agent according to any one of claims 6 to 8,
wherein the porous metal complex comprises at least one metal selected from elements belonging to Groups 2, 4, and 7 to 14 of periodic table.

10. The aldehyde removing agent according to any one of claims 6 to 9,
wherein the porous metal complex comprises at least one organic ligand containing a primary amino group, a secondary amino group, or an imino group.

11. The aldehyde removing agent according to any one of claims 6 to 10,
wherein the porous metal complex comprises at least one metal selected from elements belonging to Groups 2, 4, and 7 to 14 of periodic table and comprises one organic ligand containing a primary amino group, a secondary amino group, or an imino group.

12. The aldehyde removing agent according to any one of claims 6 to 11,
wherein the metal is at least one of Ti, Zr, Mn, Fe, Co, Ni, Cu, Zn, Al, and Si, and said organic ligand is at least one of 2-aminoterephthalic acid and 2,5-diaminoterephthalic acid.
